# EUROPEAN PATENT SPECIFICATION

(11) **EP 4 218 597 B1**
(45) Date of publication and mention of the grant of the patent: **12.02.2025**
(21) Application number: 23168360.8
(22) Date of filing: 19.06.2019
(51) Int. Cl.: A61B 8/08, A61B 8/12, A61B 8/00, A61B 17/34, A61B 10/02, A61B 17/3205, A61M 25/00

(54) **NEEDLE ASSEMBLIES WITH SAFETY ACTUATOR FOR SHARPENED NEEDLE**
NADELSYSTEME MIT SICHERHEITSBETÄTIGUNG FÜR SCHÄRFENDE NADEL
ENSEMBLES D'AIGUILLES AVEC ACTIONNEUR DE SÉCURITÉ POUR AIGUILLES AFFÛTÉES

(30) Priority: 20.06.2018 US 201862687676 P
(43) Date of publication of application: 02.08.2023
(62) Divisional of application: 19735165.3
(73) Proprietor: Boston Scientific Scimed, Inc., Maple Grove, MN 55311 (US)
(72) Inventor: JOHNSON, Austin G., Maple Grove, MN 55311 (US); O'SHAUGHNESSY, Seasmus F., Maple Grove, MN 55311 (US); DITULLIO, Jeremy, Maple Grove, MN 55311 (US); HARRIS, Colby, Maple Grove, MN 55311 (US); WALSH, Kevin, Maple Grove, MN 55311 (US); PHILLIPS-HUNGERFORD, Molly, Maple Grove, MN 55311 (US); LIEBERMAN, Nancy, Maple Grove, MN 55311 (US)
(74) Representative: Vossius & Partner Patentanwälte Rechtsanwälte mbB

(56) References cited:
- WO-A1-2015/089372
- WO-A2-2006/039199
- US-A- 5 928 200
- US-A1- 2008 091 109
- US-A1- 2014 276 051
- US-A1- 2016 374 650
- US-A1- 2017 079 519
- US-A1- 2017 303 889

## Description

### FIELD

The present disclosure relates generally to the field of medical devices. In particular, the present disclosure relates to medical systems and methods configured to shield the sharpened distal end of needles, such as exchangeable biopsy needles. The biopsy needles may be used for tissue biopsy and sampling under radial ultrasound guidance.

### BACKGROUND

As an example of systems utilizing needles, endoscopic ultrasound biopsy systems with exchangeable biopsy needles allow biopsy samples to be obtained from potentially cancerous pulmonary nodules under radial ultrasound guidance. These systems may include exchangeable needles to eliminate the need for the entire system to be removed from the patient, *e.g*., through the endoscope working channel, after each biopsy sample is taken. Exchangeable needles may improve procedure time by allowing a second biopsy sample to be taken after the previous sample is expelled for analysis, and may improve efficiency by maintaining the ultrasound image of the target nodule throughout the procedure. Once the needle is removed from the biopsy system, the exposed needle tip may pose a puncture/needle-stick risk to medical professionals in the operating room and/or technicians in the diagnostic laboratory. In addition, the tortuous anatomies within which exchangeable needle systems are deployed may result in puncture/skiving of the delivery catheter wall by the sharpened end of the biopsy needle. This skiving may result in a partial or incomplete biopsy sample and/or prevent the biopsy needle from completely retracting into the delivery catheter, thereby further promoting the potential for a puncture/needle stick.

US 2017/303889 A1 discloses a biopsy device including an ultrasonic probe, a display, an actuator, and a needle assembly. The actuator is configured to move the needle assembly in a distal direction relative to the ultrasonic probe and through the channel of the ultrasonic probe from a retracted position to a deployed position.

WO 2015/089372 A1 discloses a minimally invasive system comprising an elongate sheath and an elongate instrument slidably disposed within a lumen of the sheath. The sheath includes a flexible tube portion including a plurality of slots, a sheath element, and a rigid tube section, wherein the flexible tube portion is fixedly coupled to a distal end of the sheath element, and the rigid tube section is fixedly coupled to a distal end of the flexible tube. The lumen extends through the sheath element, the flexible tube portion, and the rigid tube section and defines a longitudinal axis of the sheath. The instrument includes a rigid distal portion adapted to slide between a retracted configuration in which the rigid distal portion is retracted within the rigid tube section and an extended configuration in which the rigid distal portion at least partially extends from the rigid tube section.

A variety of advantageous medical outcomes may therefore be realized by the systems and/or methods of the present disclosure, which provide the combined benefits of a biopsy needle assembly that may be exchangeable, may prevent or eliminate puncture or skiving of the delivery catheter during a biopsy procedure, and may shield the exposed sharpened needle tip while still allowing the biopsy sample to be expelled for analysis.

### SUMMARY

The invention is defined in claim 1 and claim 4. Further aspects and preferred embodiments are defined in the appended claims. Aspects, embodiments and examples of the present disclosure which do not fall under the scope of the appended claims do not form part of the invention and are merely provided for illustrative purposes.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIGS. 1A-1D provide perspective views of a needle assembly, according to one embodiment of the present disclosure.
FIGS. 2A-2B provide perspective views of a needle assembly, according to one embodiment of the present disclosure.
FIGS. 3A-3D provide perspective views of a needle assembly and catheter, according to one embodiment of the present disclosure.
FIG. 4 provides a front-perspective view of a catheter, according to one embodiment of the present disclosure.

### DETAILED DESCRIPTION

The present disclosure is not limited to the particular embodiments described herein. The terminology used herein is for the purpose of describing particular embodiments only, and is not intended to be limiting beyond the scope of the appended claims. Unless otherwise defined, all technical terms used herein have the same meaning as commonly understood by one of ordinary skill in the art to which the disclosure belongs.

Although embodiments of the present disclosure are described with specific reference to medical systems for acquiring biopsy samples from a pulmonary nodule under radial ultrasound guidance, it should be appreciated that such systems may be used in a variety of medical procedures where there is a need for exchangeable or single-use needle assemblies that shield the exposed needle tip while still allowing the needle tip to be accessible, including, for example, in endoscopy procedures, intravenous procedures, etc.

As used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

As used herein, the term "distal" refers to the end farthest away from the medical professional when introducing a device into a patient, while the term "proximal" refers to the end closest to the medical professional when introducing a device into a patient.

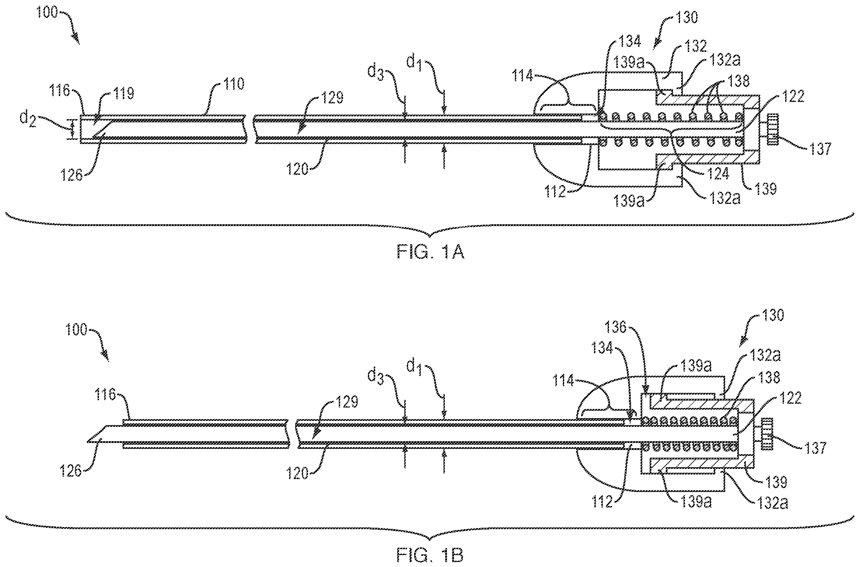

In various embodiments, the present disclosure relates to devices to shield the exposed tip of an exchangeable biopsy needle following removal from the patient. Referring to FIGS. 1A-1D, in one embodiment, a needle assembly 100 *(e.g.,* biopsy needle assembly) of the present disclosure may include a biopsy needle 120 slidably disposed within a sheath 110. The sheath 110 may include a proximal end 112, a distal end 116 and a lumen 119 extending therebetween. In various embodiments, the sheath 110 may include an outer dimension d₁ and an inner dimension d₂ *(e.g.,* of lumen 119). The needle 120 may include proximal end 122, a sharpened distal end 126 and a lumen 129 extending therebetween. In various embodiments, the biopsy needle 120 may include an outer dimension d₃ less than the inner dimension d₂ of the lumen 119 of sheath 110.

An actuation mechanism 130 may be disposed along a proximal portion of the needle assembly 100. The actuation mechanism 130 may include a housing 132, comprising a channel 134 formed within a distal portion of the housing 132 *(e.g.,* along a longitudinal axis of the housing) and a chamber 136 formed within a proximal portion of the housing 132. A spring 138 may be disposed within, and extend along a longitudinal length of, the chamber 136. A push button 139 may be engaged with the proximal portion of the housing 132 and configured to move between a plurality of distally advanced and proximally retracted positions within the chamber 136. In various embodiments, the proximal portion of the housing 132 may include one or more surface features 132a configured to engage one or more corresponding surface features 139a formed along the distal portion of the push button 139. The surface features 132a, 139a may be configured to contact each other to maintain the push button 139 within the housing 132 *(e.g.,* prevent separation) when the push button 139 is in the proximally retracted configuration. **In** various embodiments, the surface features 132a, 139a may include one or more inward and outward extending tabs (respectively) disposed in various patterns (including a fully circumferential patterns) along the respective proximal and distal ends of the housing 132 and push button 139. In addition, or alternatively, the housing 132 and push button 139 may include a variety of configurations to prevent unintended separation, including, for example, corresponding thread or luer-lock connector configurations, and the like.

A proximal portion 114 of the sheath 110 may be immovably attached or affixed to the distal portion of the housing 132. In various embodiments, the proximal portion 114 of the sheath 110 may be bonded, adhered and/or integrally formed with at least a portion of the channel 134 of housing 132. A proximal portion 124 of the needle 120 may extend proximally beyond the proximal end 112 of the sheath 110, and through the spring 138 within the chamber 136, such that the proximal end 122 of the needle 120 contacts (or is adjacent to) an inner surface of the push button 139. In various embodiments, the proximal end 122 of the needle 120 may be attached to a stylet 137 extending through an opening (not shown) formed within a proximal end of the push button 139. The stylet 137 may extend through all, or a portion, of the lumen 129 of the needle 120. The stylet may obstruct or occlude the lumen 129, *e.g.,* as the needle is advanced through narrow and tortuous body passages. The stylet 137 may be removed to provide access to the lumen 129, *e.g.,* to expel or aspirate a biopsy sample, or to inject a fluid into or around the target pulmonary nodule.

In various embodiments, the push button 139 may be distally advanced/depressed, *e.g.,* under the force applied by a thumb and forefinger of a user, to compress the spring 138 within the chamber 136, thereby distally advancing the needle 120 relative to the sheath 110 such that the sharpened distal end 126 of the needle 120 moves from a first position (*e.g*., shielded, retracted or protected position) within the lumen 119 proximal to the distal end 116 the sheath 110 (FIG. 1A), to a second position (*e.g.,* exposed, extended, unprotected) distally beyond (*e.g.*, positioned distal to) the distal end 116 of the sheath 110 (FIG. 1B). Similarly, the push button 139 may be proximally retracted/released, *e.g*., by removing or reducing the distal force exerted by the user, such that the spring 138 returns to the relaxed (*e.g*., non-compressed) position, thereby retracting the sharpened distal end 126 of the needle 120 into the lumen 119 of the sheath 110.

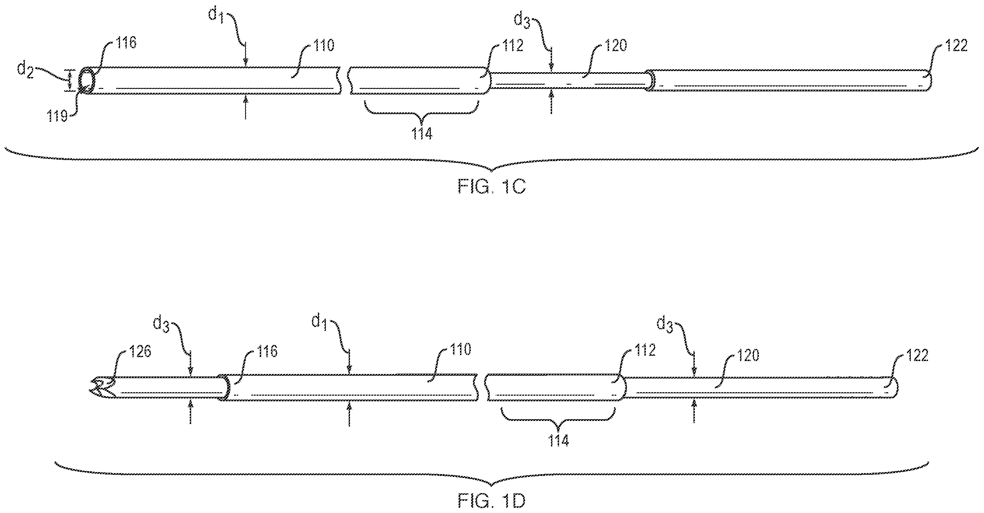

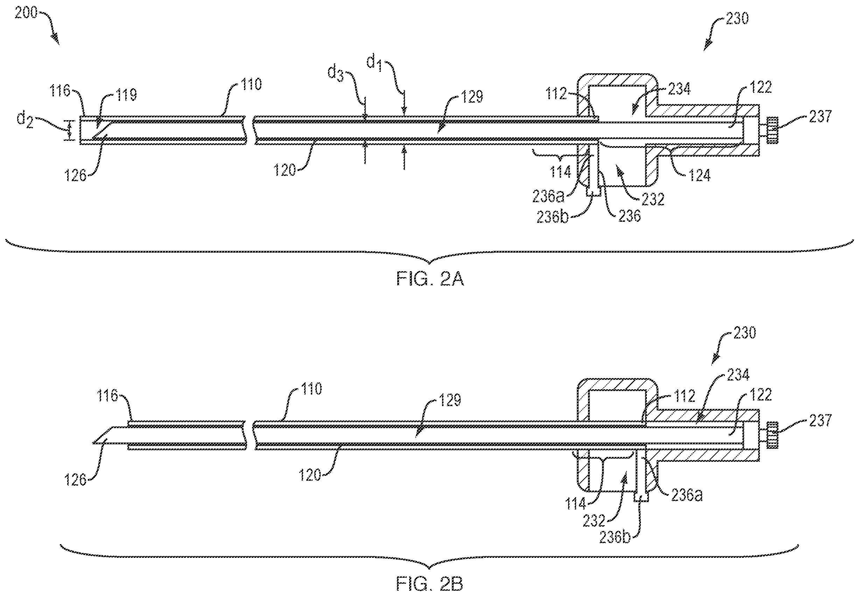

Referring to FIGS. 2A and 2B, in one embodiment, a needle assembly 200 *(e.g.,* biopsy needle assembly) of the present disclosure may include a sheath 110 and needle 120, *e.g.,* as depicted in FIGS. 1C and 1D, with the exception that the sheath 110 is slidably disposed around at least a portion of the needle 120. The sheath 110 may include a proximal end 112, a distal end 116 and a lumen 119 extending therebetween. The sheath 110 may include an outer dimension d₁ and an inner dimension d₂ *(e.g.,* of lumen 119). The needle 120 may include a proximal end 122, a sharpened distal end 126 and a lumen 129 extending therebetween. The biopsy needle may include an outer dimension d₃ less than the inner dimension d₂ of the lumen 119 of sheath 110.

An actuation mechanism 230 may be disposed along a proximal portion of the needle assembly 200. The actuation mechanism 230 may include a housing 232, a channel 234 may extend through/along a length (*e.g.,* longitudinal axis) of the housing 232 and a slot or opening 233 may extend through a wall of the housing 232 in communication with (*e.g*., opening into) a distal portion of the channel 234. An arm 236 (*e.g.,* slider arm, trigger, finger grip, etc.) may extend through the slot 233 of the housing 232 such that a first end 236a of the arm 236 extends into, or is substantially coextensive with, the channel 234 and a second end 236b of the arm extends outside the housing 232.

A proximal portion 114 of the sheath 110 may be slidably/movably disposed within the distal portion of the channel 234. The first end 236a of the arm 236 may be attached to, or integrally formed with, the distal end 116 of the sheath 110. A proximal portion 124 of the needle 120 may extend proximally beyond the proximal end 112 of the sheath 110 and through the remaining (*e.g.,* proximal portion) of the channel 234 such that the proximal end 122 of the needle 120 contacts (or is adjacent to) an inner surface of the housing 232. **In** various embodiments, the proximal end 122 of the needle 120 may be attached to a stylet 237 extending through an opening (not shown) formed within a proximal end of the housing 232. As above, the stylet 237 may extend through all, or a portion, of the lumen 129 of the needle 120. The stylet may obstruct or occlude the lumen 129, *e.g.,* as the needle is advanced through narrow and tortuous body passages. The stylet 137 may be removed to provide access to the lumen 129, *e.g.,* to expel or aspirate a biopsy sample, or to inject a fluid into or around the target pulmonary nodule.

**In** various embodiments, the arm 236 may be proximally retracted along the slot 233, *e.g.,* under the force applied by a thumb and forefinger of a user, thereby proximally retracting the sheath 110 relative to the needle 120 such that the sharpened distal end 126 of the needle 120 moves from a first position (*e.g*., shielded, retracted or protected position) within the lumen 119 proximal to the distal end 116 the sheath 110 (FIG. 2A), to a second position *(e.g.,* exposed, extended, unprotected) distally beyond the distal end 116 of the sheath 110 (FIG. 2B). Similarly, the arm 236 may be distally advanced along the slot 233 such that the sheath 110 moves along the needle 120 to position/reposition the distal end 116 of the sheath 110 distally beyond the sharpened distal end 126 of the needle 120.

In various embodiments, a spring (not shown) may be disposed within a portion of the channel 234 proximally beyond the proximal end of the sheath 110, and with the proximal portion of the needle 120 extending through the spring, as discussed above. As the arm 236 is proximally retracted along the slot 233, the spring may compress within the channel 234 to expose the sharpened distal end 126 of the needle 120. The arm 236 and sheath 110 may then return to the first position by partially or fully releasing the pressure exerted on the arm 236 such that the spring 138 returns to the relaxed *(e.g.,* non-compressed) position.

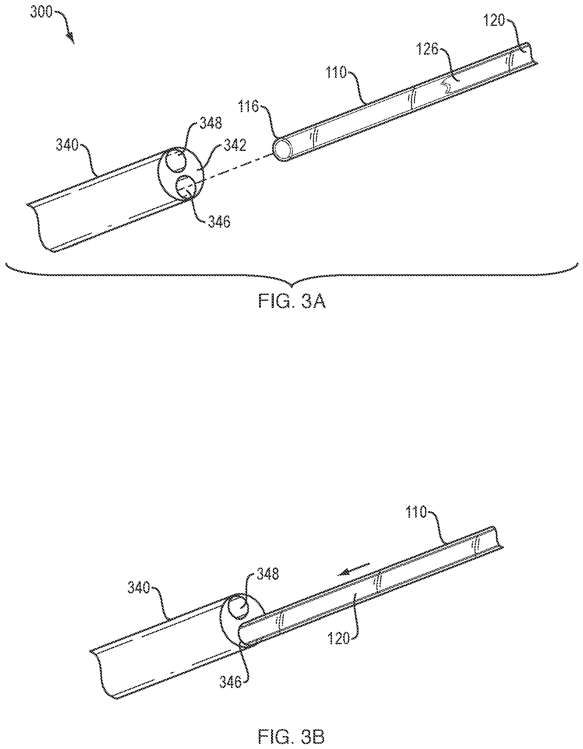

Referring to FIGS. 3A-3D, in one embodiment, a system 300 of the present disclosure may include a needle assembly 100, 200 slidably disposed within a dual-lumen catheter 340. In various embodiments, the dual-lumen catheter 340 may include a proximal end 342, a distal end 344, a first lumen 346 and a second lumen 348 extending therebetween. The first lumen 346 may include an inner dimension d₄ sized and configured to receive the outer dimension d₁ of the sheath 110 of the needle assembly 100, 200. The second lumen 348 may include an inner dimension d₅ sized and configured to receive a radial ultrasound probe (not shown).

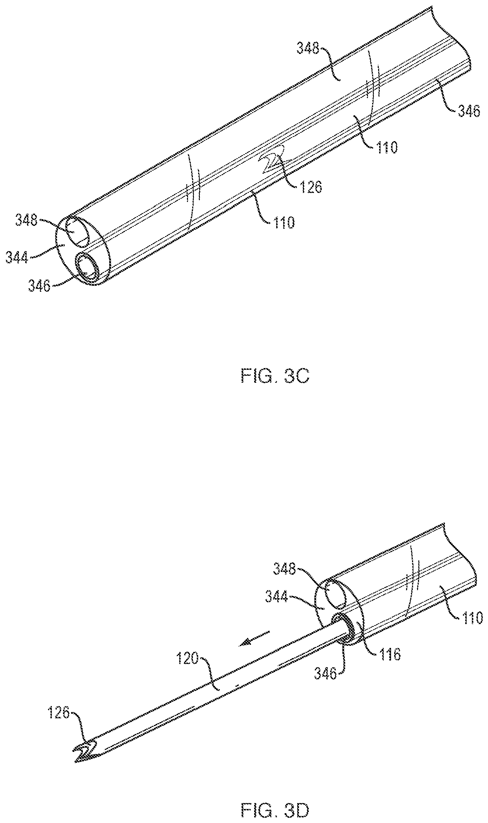

Referring to FIG. 3C, the sheath 110 and needle 120 of the needle assembly 100, 200 may be advanced in the first position (FIGS. 1A, 2A) through the first lumen 346 to position the distal end 116 of the sheath 110 adjacent to (or substantially coextensive with) the distal end 344 of the dual-lumen catheter 340.

Referring to FIG. 3D, in one embodiment, the needle assembly 100 may be moved from the first to second position, *e.g.,* by distally advancing push button 139 (as discussed above) to advance the sharpened distal end 126 of the needle 120 distally beyond the respective distal ends 116, 344 of the sheath 110 and dual-lumen catheter 340. Still referring to FIG. 3D, in another embodiment, the needle assembly 200 may be moved from the first to second position, *e.g.,* by proximally retracting the arm 236 and sheath 110 (as discussed above), to such that the sharpened distal end 126 of the needle 120 extends distally beyond the distal end 116 of the sheath 110 within the first lumen 346 of the dual-lumen catheter 340. The needle assembly 200, including the exposed sharpened distal end 126 of the needle 120 may then be further distally advanced through the first lumen 346 while maintaining the arm 236 and sheath 110 in the second position, such that the sharpened distal end 126 of the needle 120 distally beyond the distal end 344 of the dual-lumen catheter 340.

In use, and by way of example, a needle biopsy system 300 of the present disclosure may be inserted into a pulmonary passage of a patient through a working channel of an endoscope and adjacent to a target pulmonary nodule. The stylet 137 may then be removed from the lumen of the biopsy needle 120, and the sharpened distal end 126 of the needle 120 advanced distally beyond the distal end 344 of the dual-lumen catheter 340 into the target pulmonary nodule (as discussed above). The user may distally deploy the needle 120 into the target pulmonary nodule as many times as necessary to acquire sufficient tissue for analysis. When the user determines that the biopsy needle contains a sufficient amount of tissue from the target pulmonary nodule, the needle assembly 100, 200 may be returned to the first position (*e.g*., with the sharpened distal end 126 positioned proximal to the distal end 116 of the sheath 110), and the needle assembly 100, 200 proximally withdrawn from the first lumen 346 of the dual-lumen catheter 340. The sharpened distal end 126 of the needle is therefore shielded from the user and/or ancillary personnel immediately upon removal from the sheath 110. In various embodiments, the needle assembly 100, 200 may be removed from within the first lumen 346 of the dual-lumen catheter 340 without changing the position of the dual-lumen catheter 340 and radial ultrasound probe relative to the target pulmonary nodule.

In various embodiments, the tissue sample may then be expelled from the needle assembly while the sharpened distal end 126 remains safely housed within the sheath 110. Alternatively, the sharpened distal end 126 of the needle 120 may be advanced distally beyond the distal end 116 of the sheath 110 prior to expelling the tissue sample. The needle assembly 100, 200 may then be proximally retracted into the sheath 110, and the needle assembly 100, 200 re-introduced through the first lumen 346 of the dual-lumen catheter 340 to acquire an additional biopsy sample from the same, or a different, pulmonary nodule. Alternatively, one or more different needle assemblies 100, 200 may be introduced through the first lumen 346 of the dual-lumen catheter 340 while the biopsy sample is being expelled from the original needle assembly 100, 200. The ability to obtain multiple additional biopsy samples from the same or different pulmonary nodule while the biopsy sample is expelled from the original *(e.g.,* first needle assembly) may further improve procedure time, efficiency and accurate/reliable diagnostic results.

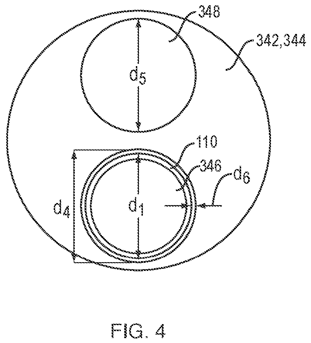

In various embodiments, the respective inner and outer dimensions (d₁-d₄) of the sheath 110, needle 120 and first lumen 346 may be configured to minimize or prevent puncture or skiving of the sheath 110 and/or dual-lumen catheter 340 by the sharpened distal end 126 of the needle 120, *e.g.,* within narrow and tortuous body passages in which the needle biopsy system 300 may be bent or torqued at a variety of angles during biopsy sample collection. Referring to FIG. 4, in one embodiment, the inner dimension d₄ of the first lumen 346 and the outer dimension d₁ of the sheath 110 may be separated by a distance d₆ (*e.g*., approximately 0.0002 inch) configured to allow the needle assembly 100, 200 to move smoothly (*e.g*., with low or minimal friction) within the first lumen 346 with minimal lateral movement of the needle assembly 100, 200, *e.g.,* which might contribute to puncture or skiving of the sheath 110 and/or dual-lumen catheter 340 during biopsy sample collection or expulsion.

In various embodiments, the sheath 110 may comprise a variety of low friction materials along all or a portion of the length of the sheath to minimize friction between the first lumen 346 of the dual-lumen catheter 340 and/or biopsy needle 120. The sheath 110 may be formed entirely from these low friction materials, or provided as inner and/or outer coatings of the sheath. Non-limiting examples of low-friction materials includes, Pebax, loaded Pebax, BaSO₄, PTFE, Mediglide^{™}, Polymide, PEEK and glass-fiber reinforced PEEK. In addition, or alternatively, the first and/or second lumens 346, 348 of the dual-lumen catheter 340 may include a coating or liner of any of these low friction materials. In addition, or alternatively, at least a portion of the sheath 110 may comprise an optically translucent material (*e.g.,* glass, plastic, rubber, etc.) to allow a user to visualize the sharpened end the needle 120 and/or biopsy sample disposed therein.

In various embodiments, the sharpened distal end 126 of the needle 120 may include a needle grind configured to further reduce the potential for puncture or skiving of the sheath 110 and/or dual-lumen catheter 340. For example, the needle 120 may include a three-point "Francine" needle grind, in which the sharpened points of the needle 120 extend slightly inward toward the lumen of the needle to further minimize the potential for puncture or skiving of the sheath or dual-lumen catheter. In addition to reducing friction, these low friction materials and/or coatings may further reduce the likelihood or ability of the sharpened distal end of the needle to puncture or skive the sheath and/or dual-lumen catheter. To further reduce the puncture or skiving of the sheath and/or dual-lumen catheter, in various embodiments, the needle 120 may be formed from or otherwise include a variety of flexible and/or kink-resistant materials (e.g., Nitnol, etc.) along all, or a portion of, the length of the needle 120.

In various embodiments, the sheath 110 may be formed from or include a variety of metallic, plastic, fiber or composite materials, or combinations thereof, along all or a portion of the length of the sheath. Such materials may provide the requisite mechanical strength for insertion of the sheath 110 into, and removal from, the tortuous path of the dual-lumen catheter 340 within the body, and/or minimize the potential for skiving by the sharpened distal end 126 of the needle 120. As will be understood by those of skill in the art, such metallic, plastic, fiber and/or composite materials may also reduce or eliminate the tendency of the sheath 110 to compress and/or stretch during insertion into, and removal from the dual-lumen catheter 340.

In addition, or alternatively, one or more slots or grooves may be included *(e.g.,* laser slotted) along all or various portions of the length of the sheath 110 and/or dual-lumen catheter 340 to further increase the flexibility of the needle biopsy system 300, *e.g*., within tortuous body passages. In various embodiments, the sheath 110 is not limited to unitary or solid configurations, but may be formed from a hypotube, coil and/or braid along all or a portion of the length of the sheath.

In various embodiments, the sheath 110 and first lumen 346 of the dual-lumen catheter 340 are not limited to circular configurations, but may include a variety of cross-sectional shapes and/or geometries, including, for example, elliptical, oval or star-shaped geometries. In various embodiments, these geometries may minimize the surface area of the sheath 110 in contact with the inner wall of first lumen 346 the dual-lumen catheter 340, thereby further reducing friction and the potential for puncture or skiving by the sharpened distal end 126 of the needle 120.

The needle assemblies 100, 200 of the present disclosure are in no way limited to systems that include dual-lumen catheters, but may be employed in a variety of single or multi-lumen catheters, including by way of non-limiting example, triple-lumen catheters, quadruple-lumen catheters and the like. In addition, the systems of the present disclosure are in no way limited to use with radial ultrasound probes, but may include a variety of imaging modalities, including, for example, linear ultrasound probes, etc. Alternatively, the systems of the present disclosure may be used in standalone procedures that do not include any form of imaging modality or probe.

All of the devices disclosed and claimed herein can be made without undue experimentation in light of the present disclosure. While the devices of this disclosure have been described in terms of preferred embodiments, it may be apparent to those of skill in the art that variations can be applied to the devices described herein without departing from the scope of the disclosure. All such similar substitutes and modifications apparent to those skilled in the art are deemed to be within the scope of the disclosure as defined by the appended claims.

## Claims

1. A needle assembly (100), comprising:
a sheath (110) comprising a proximal end (112), a distal end (116) and a lumen (119) extending therebetween;
a needle (120) disposed within the lumen of the sheath, the needle comprising a proximal (122) end and a sharpened distal end (126); and
an actuation mechanism (130) disposed along a proximal portion of the needle assembly, the actuation mechanism (130) comprising a housing (132), a channel (134) formed within a distal portion of the housing (132), a spring (138) disposed within a chamber (136) of the housing (132) and a push button (139) engaged with a proximal portion of the housing (132), the push button (139) configured to move from a first position to a second position within the housing (132);
wherein a proximal portion (114) of the sheath (110) is immovably disposed within the channel (134) of the housing;
wherein a proximal portion of the needle (120) extends through the spring (138);
wherein the needle (120) moves in a distal direction relative to the sheath (110) when the push button (139) moves distally within the chamber (136) and the needle (120) moves in a proximal direction relative to the sheath (110) when the push button (139) moves proximally within the chamber (136).

2. The needle assembly (100) of claim 1, wherein the proximal end of the housing (132) includes a surface feature configured to engage a corresponding surface feature on a distal end of the push button (139).

3. The needle assembly (100) of claim 1, wherein an inner surface, or outer surface, or both inner and outer surface, of the sheath (110) includes a low friction material.

4. A needle assembly (200), comprising:
a sheath (110) comprising a proximal end (112), a distal end (116) and a lumen (119) extending therebetween;
a needle (120) disposed within the lumen of the sheath, the needle (120) comprising a proximal end (122) and a sharpened distal end (126); and
an actuation mechanism (230) disposed along a proximal portion of the needle assembly, the actuation mechanism (230) comprising a housing (232), a channel (234) extending through a portion of the housing (232), a slot (233) extending through a wall of the housing and an arm (236) extending through the slot (233);
wherein a proximal portion (114) of the sheath (110) is slidably disposed within a distal portion of the channel (234);
wherein a proximal portion of the needle (120) extends through the sheath (110) and through a length of the channel (234);
wherein a first end (236a) of the arm (236) is attached to the proximal end of the sheath (110), and a second end (236b) of the arm (236) extends outside of the actuation mechanism (230); and
wherein the sheath (110) moves in a proximal direction relative to the needle (120) when the arm (236) moves proximally along the slot (233), and moves in a distal direction relative to the needle (120) when the arm moves distally along the slot (233).

5. The needle assembly (200) of claim 4, wherein the arm is configured to move between a first position and a second position along the slot.

6. The needle assembly (200) of claim 5, wherein the sharpened distal end of the needle (120) is positioned proximal to the distal end of the sheath (110) when the arm (236) is in the first position, and the sharpened distal end of the needle (120) is positioned distal to the distal end of the sheath (110) when the arm (236) is in the second position.

7. The needle assembly (200) of any of claims 4-6, wherein an inner surface, or outer surface, or both the inner and outer surface, of the sheath (110) includes a low friction material.

8. The needle assembly (100, 200) of any of claims 1-7, wherein the sharpened distal end of the needle (120) includes a three-point needle grind.

9. The needle assembly (100, 200) of any of claims 1-7, wherein at least a portion of the sheath (110) includes an optically translucent material.

10. The needle assembly (100, 200) of any of claims 1-9, further comprising one or more grooves formed along a length of the sheath (110).

11. The needle assembly (100, 200) of any of claims 1-10, wherein a cross-section of the sheath (110) includes a circular, elliptical, oval or star-shaped geometry.

## Patentansprüche

1. Nadelanordnung (100), die aufweist:
eine Hülse (110) mit einem proximalen Ende (112), einem distalen Ende (116) und einem sich dazwischen erstreckenden Lumen (119);
eine Nadel (120), die im Lumen der Hülse angeordnet ist, wobei die Nadel ein proximales Ende (122) und ein geschärftes distales Ende (126) aufweist; und
einen Betätigungsmechanismus (130), der entlang eines proximalen Abschnitts der Nadelanordnung angeordnet ist, wobei der Betätigungsmechanismus (130) aufweist: ein Gehäuse (132), einen Kanal (134), der in einem distalen Abschnitt des Gehäuses (132) gebildet ist, eine Feder (138), die in einer Kammer (136) des Gehäuses (132) angeordnet ist, und einen Druckknopf (139), der einen Eingriff mit einem proximalen Abschnitt des Gehäuses (132) herstellt, wobei der Druckknopf (139) so konfiguriert ist, dass er sich aus einer ersten Position in eine zweite Position im Gehäuse (132) bewegt;
wobei ein proximaler Abschnitt (114) der Hülse (110) im Kanal (134) des Gehäuses unbeweglich angeordnet ist;
wobei sich ein proximaler Abschnitt der Nadel (120) durch die Feder (138) erstreckt;
wobei sich die Nadel (120) in distaler Richtung relativ zur Hülse (110) bewegt, wenn sich der Druckknopf (139) in der Kammer (136) distal bewegt, und sich die Nadel (120) in proximaler Richtung relativ zur Hülse (110) bewegt, wenn sich der Druckknopf (139) in der Kammer (136) proximal bewegt.

2. Nadelanordnung (100) nach Anspruch 1, wobei das proximale Ende des Gehäuses (132) ein Oberflächenmerkmal aufweist, das so konfiguriert ist, dass es einen Eingriff mit einem entsprechenden Oberflächenmerkmal auf einem distalen Ende des Druckknopfs (139) herstellt.

3. Nadelanordnung (100) nach Anspruch 1, wobei eine Innenfläche oder Außenfläche oder sowohl die Innen- als auch die Außenfläche der Hülse (110) ein reibungsarmes Material aufweist.

4. Nadelanordnung (200), die aufweist:
eine Hülse (110) mit einem proximalen Ende (112), einem distalen Ende (116) und einem sich dazwischen erstreckenden Lumen (119);
eine Nadel (120), die im Lumen der Hülse angeordnet ist, wobei die Nadel (120) ein proximales Ende (122) und ein geschärftes distales Ende (126) aufweist; und
einen Betätigungsmechanismus (230), der entlang eines proximalen Abschnitts der Nadelanordnung angeordnet ist, wobei der Betätigungsmechanismus (230) aufweist: ein Gehäuse (232), einen Kanal (234), der sich durch einen Abschnitt des Gehäuses (232) erstreckt, einen Schlitz (233), der sich durch eine Wand des Gehäuses erstreckt, und einen Arm (236), der sich durch den Schlitz (233) erstreckt;
wobei ein proximaler Abschnitt (114) der Hülse (110) in einem distalen Abschnitt des Kanals (234) verschiebbar angeordnet ist;
wobei sich ein proximaler Abschnitt der Nadel (120) durch die Hülse (110) und durch eine Länge des Kanals (234) erstreckt;
wobei ein erstes Ende (236a) des Arms (236) am proximalen Ende der Hülse (110) angebracht ist und sich ein zweites Ende (236b) des Arms (236) außerhalb des Betätigungsmechanismus (230) erstreckt; und
wobei sich die Hülse (110) in proximaler Richtung relativ zur Nadel (120) bewegt, wenn sich der Arm (236) entlang des Schlitzes (233) proximal bewegt, und sich in distaler Richtung relativ zur Nadel (120) bewegt, wenn sich der Arm entlang des Schlitzes (233) distal bewegt.

5. Nadelanordnung (200) nach Anspruch 4, wobei der Arm so konfiguriert ist, dass er sich zwischen einer ersten Position und einer zweiten Position entlang des Schlitzes bewegt.

6. Nadelanordnung (200) nach Anspruch 5, wobei das geschärfte distale Ende der Nadel (120) proximal zum distalen Ende der Hülse (110) positioniert ist, wenn sich der Arm (236) in der ersten Position befindet, und das geschärfte distale Ende der Nadel (120) distal zum distalen Ende der Hülse (110) positioniert ist, wenn sich der Arm (236) in der zweiten Position befindet.

7. Nadelanordnung (200) nach einem der Ansprüche 4 bis 6, wobei eine Innenfläche oder Außenfläche oder sowohl die Innen- als auch die Außenfläche der Hülse (110) ein reibungsarmes Material aufweist.

8. Nadelanordnung (100, 200) nach einem der Ansprüche 1 bis 7, wobei das geschärfte distale Ende der Nadel (120) einen Dreipunkt-Nadelanschliff aufweist.

9. Nadelanordnung (100, 200) nach einem der Ansprüche 1 bis 7, wobei mindestens ein Abschnitt der Hülse (110) ein optisch durchscheinendes Material aufweist.

10. Nadelanordnung (100, 200) nach einem der Ansprüche 1 bis 9, die ferner eine oder mehrere Nuten aufweist, die über eine Länge der Hülse (110) gebildet sind.

11. Nadelanordnung (100, 200) nach einem der Ansprüche 1 bis 10, wobei ein Querschnitt der Hülse (110) eine kreisförmige, elliptische, ovale oder sternförmige Geometrie aufweist.

## Revendications

1. Ensemble d'aiguille (100), comprenant:
une gaine (110) comprenant une extrémité proximale (112), une extrémité distale (116) et une lumière (119) s'étendant entre celles-ci;
une aiguille (120) disposée à l'intérieur de la lumière de la gaine, l'aiguille comprenant une extrémité proximale (122) et une extrémité distale aiguisée (126); et
un mécanisme d'actionnement (130) disposé le long d'une partie proximale de l'ensemble d'aiguille, le mécanisme d'actionnement (130) comprenant un boîtier (132), un canal (134) formé à l'intérieur d'une partie distale du boîtier (132), un ressort (138) disposé à l'intérieur d'une chambre (136) du boîtier (132) et un bouton-poussoir (139) en prise avec une partie proximale du boîtier (132), le bouton-poussoir (139) étant configuré pour se déplacer d'une première position à une seconde position à l'intérieur du boîtier (132);
dans lequel une partie proximale (114) de la gaine (110) est disposée de manière immobile à l'intérieur du canal (134) du boîtier;
dans lequel une partie proximale de l'aiguille (120) s'étend à travers le ressort (138);
dans lequel l'aiguille (120) se déplace dans une direction distale par rapport à la gaine (110) lorsque le bouton-poussoir (139) se déplace de manière distale à l'intérieur de la chambre (136) et l'aiguille (120) se déplace dans une direction proximale par rapport à la gaine (110) lorsque le bouton-poussoir (139) se déplace de manière proximale à l'intérieur de la chambre (136).

2. Ensemble d'aiguille (100) selon la revendication 1, dans lequel l'extrémité proximale du boîtier (132) inclut une caractéristique de surface configurée pour venir en prise avec une caractéristique de surface correspondante sur une extrémité distale du bouton-poussoir (139).

3. Ensemble d'aiguille (100) selon la revendication 1, dans lequel une surface interne, ou une surface externe, ou une surface interne et une surface externe, de la gaine (110), incluent l'une et l'autre un matériau à faible frottement.

4. Ensemble d'aiguille (200), comprenant:
une gaine (110) comprenant une extrémité proximale (112), une extrémité distale (116) et une lumière (119) s'étendant entre celles-ci;
une aiguille (120) disposée à l'intérieur de la lumière de la gaine, l'aiguille (120) comprenant une extrémité proximale (122) et une extrémité distale aiguisée (126); et
un mécanisme d'actionnement (230) disposé le long d'une partie proximale de l'ensemble d'aiguille, le mécanisme d'actionnement (230) comprenant un boîtier (232), un canal (234) s'étendant à travers une partie du boîtier (232), une fente (233) s'étendant à travers une paroi du boîtier et un bras (236) s'étendant à travers la fente (233);
dans lequel une partie proximale (114) de la gaine (110) est disposée de manière coulissante à l'intérieur d'une partie distale du canal (234);
dans lequel une partie proximale de l'aiguille (120) s'étend à travers la gaine (110) et à travers une longueur du canal (234);
dans lequel une première extrémité (236a) du bras (236) est fixée à l'extrémité proximale de la gaine (110), et une seconde extrémité (236b) du bras (236) s'étend à l'extérieur du mécanisme d'actionnement (230); et
dans lequel la gaine (110) se déplace dans une direction proximale par rapport à l'aiguille (120) lorsque le bras (236) se déplace de manière proximale le long de la fente (233), et se déplace dans une direction distale par rapport à l'aiguille (120) lorsque le bras se déplace de manière distale le long de la fente (233).

5. Ensemble d'aiguille (200) selon la revendication 4, dans lequel le bras est configuré pour se déplacer entre une première position et une seconde position le long de la fente.

6. Ensemble d'aiguille (200) selon la revendication 5, dans lequel l'extrémité distale aiguisée de l'aiguille (120) est positionnée de manière proximale par rapport à l'extrémité distale de la gaine (110) lorsque le bras (236) est dans la première position, et l'extrémité distale aiguisée de l'aiguille (120) est positionnée de manière distale par rapport à l'extrémité distale de la gaine (110) lorsque le bras (236) est dans la seconde position.

7. Ensemble d'aiguille (200) selon l'une quelconque des revendications 4 à 6, dans lequel une surface interne, ou une surface externe, ou la surface interne et la surface externe, de la gaine (110), incluent l'une et l'autre un matériau à faible frottement.

8. Ensemble d'aiguille (100, 200) selon l'une quelconque des revendications 1 à 7, dans lequel l'extrémité distale aiguisée de l'aiguille (120) inclut un meulage d'aiguille à trois pointes.

9. Ensemble d'aiguille (100, 200) selon l'une quelconque des revendications 1 à 7, dans lequel au moins une partie de la gaine (110) inclut un matériau optiquement translucide.

10. Ensemble d'aiguille (100, 200) selon l'une quelconque des revendications 1 à 9, comprenant en outre une ou plusieurs rainures formées le long d'une longueur de la gaine (110).

11. Ensemble d'aiguille (100, 200) selon l'une quelconque des revendications 1 à 10, dans lequel une section transversale de la gaine (110) inclut une géométrie circulaire, elliptique, ovale ou en forme d'étoile.
